# EUROPEAN PATENT SPECIFICATION

(11) **EP 4 157 232 B1**
(45) Date of publication and mention of the grant of the patent: **20.05.2026**
(21) Application number: 21814238.8
(22) Date of filing: 28.05.2021
(51) Int. Cl.: A61L 26/00, A61L 27/20, A61L 27/26, A61L 27/54

(54) **DEVICES COMPRISING HYALURONIC ACID AND SILK FIBROIN**
VORRICHTUNGEN MIT HYALURONSÄURE UND SEIDENFIBROIN
DISPOSITIFS COMPRENANT DE L'ACIDE HYALURONIQUE ET DE LA FIBROÏNE DE SOIE

(30) Priority: 28.05.2020 US 202063030952 P
(43) Date of publication of application: 05.04.2023
(73) Proprietor: University of Montana, Missoula MT 59812 (US)
(72) Inventor: SERBAN, Monica, Missoula, Montana 59812 (US)
(74) Representative: Guardian IP Consulting I/S
(86) International application number: PCT/US2021/034805
(87) International publication number: WO 2021/243182

(56) References cited:
- CN-B- 103 469 351
- US-A1- 2005 260 753
- US-A1- 2009 232 963
- US-A1- 2015 238 617
- US-A1- 2015 306 237
- US-A1- 2016 279 401
- US-A1- 2017 027 679
- US-A1- 2020 139 006
- US-B2- 10 245 306
- US-B2- 8 975 073
- US-B2- 9 566 365

## Description

### CROSS-REFERENCE TO RELATED APPLICATIONS

This application claims the benefit of U.S. Provisional Application No. 63/030,952, filed May 28, 2020.

### FIELD

The present invention relates to biocompatible and bioresorbable devices for tissue repair and regeneration and delivery of an active agent across a biological barrier comprising silk fibroin and hyaluronic acid.

### BACKGROUND

Repair and regeneration of damaged and diseased tissues is one of the most complex biological processes that occurs in living organisms. Poor wound healing after trauma, surgery, acute illness, or chronic disease conditions affects millions of humans worldwide each year and is the consequence of poorly regulated elements of the healthy tissue repair response, including inflammation, angiogenesis, matrix deposition, and cell recruitment. Evidence of treating damaged and diseased tissue has found even in the very early stages of human civilization, e.g., by making plasters and bandaging the tissue. Modern advancements in tissue repair include more effective treatment strategies including wound debridement, compression bandaging, wound dressings, hyperbaric oxygen therapy, ultrasound, electrical stimulation, and implantable controlled release drug depots. However, most of these methods rarely interact with endogenous tissue repair and regeneration mechanisms and some require expensive equipment or devices only suitable for hospital settings.

US 2016/279401 describes a device for delivering an active agent, e.g. a vitamin or antioxidant, to the skin. The device comprises a substrate and an arrangement of microneedles projecting from the substrate. The microneedles comprise a mixture of a polymeric material, preferably crosslinked hyaluronic acid, and an active agent beneficial to the skin. CN 103 469 351 describes a membrane formed from electrospun composite nanofibers, said nanofibers comprising silk fibroin and hyaluronic acid, and being loaded with vitamin C.

### SUMMARY

The scope of this invention Is defined by the claims. Any references In the description to methods of treatment refer to the devices of the present invention for use In a method for treatment of the human (or animal) body by therapy. Any "embodiment" or "example" which is disclosed in the description but is not covered by the claims should be considered as presented for illustrative purpose only.

Provided herein are biocompatible devices comprising a base layer comprising a combination of silk fibroin and hyaluronic acid, wherein the hyaluronic acid is conjugated with an antioxidant. In some embodiments, the base layer is from about 0.5 mm to about 4 mm thick. According to the claimed invention, the device is 0.5 mm to 5 mm thick and has a flexibility consistent with topical application on an exterior tissue surface such as skin. The device Is treated with 90% ethanol (EtOH) to induce physical crosslinking.

The antioxidant can be selected from methionine, cysteine, tryptophan, tyrosine, homocysteine, Vitamin A, Vitamin C, Vitamin E, or a combination thereof.

In some embodiments, the silk fibroin is chemically or chemoenzymatically modified (e.g., carboxylated silk fibroin, hydroxylated silk fibroin, methylated silk fibroin, diazonium coupled silk fibroin, methacrylated silk fibroin, a silk fibroin modified at a tyrosine, hydroxy, or amine group, or combinations thereof).

In some embodiments, the base layer comprises from about 0.1% to about 20% w/v silk fibroin. In some embodiments, the base layer comprises from about 0.1% to about 10% w/v hyaluronic acid.

In some embodiments, the base layer further comprises a bioactive macromolecule selected from the group consisting of collagen, gelatin, fibrinogen, elastin, laminin, keratin, actin, myosin, cellulose, amylose, dextran, chitin, glycosaminoglycans, and combinations thereof.

In some embodiments, the device further comprises a microneedle layer comprising a plurality of microneedles comprising a biocompatible material (e.g., silk fibroin, hyaluronic acid, polyglycolic acid (PGA), polylactic acid (PLA), polydioxanone (PDS), polycaprolactone (PCL), poly(lactic-co-glycolic acid) PLGA, polyhydroxyalkanoate (PHA), and conjugates, variants, and combinations thereof).

In some embodiments, the device is self-adherent In some embodiments, the device further comprises an adhesive layer.

In some embodiments, the device further comprises an active agent. In some embodiments, the active agent is applied to an external surface of the microneedles. In some embodiments, the active agent is embedded throughout the base layer, the microneedle layer, or a combination thereof.

Also provided herein is the disclosed device according to the claimed invention for use in methods of regenerating and repairing a tissue and for use in methods of delivery of an active agent across a biological barrier, said methods comprising applying the disclosed devices to a tissue or biological barrier of interest

Further disclosed are methods of fabricating the disclosed devices. In some embodiments, the methods comprise:
preparing a solution of the base layer components comprising a combination of silk fibroin and hyaluronic acid, wherein the hyaluronic acid is conjugated with an antioxidant; filling a base layer mold with the solution of the base layer components; removing a solidified base layer from the base layer mold; crosslinking the base layer by treatment with 90% ethanol;
and optionally further comprises the steps of filling a microneedle layer mold with a solution of the biocompatible material;
removing a solidified microneedle layer from the base layer mold; applying a solution of silk fibroin to one side of the base layer and or the microneedle layer; joining the base layer to the microneedle layer; and adding an active agent. In some embodiments, the methods comprise preparing a solution of a combination of silk fibroin and hyaluronic acid, wherein the hyaluronic acid is conjugated with an antioxidant; filling a combined microneedle-base layer mold with the solution; removing a solidified microneedle-base layer from the combined microneedle-base layer mold; crosslinking the solidified microneedle-base layer by treatment with 90% ethanol; and
adding an active agent.

Other aspects and embodiments of the disclosure will be apparent in light of the following detailed description and accompanying figures.

### BRIEF DESCRIPTION OF THE DRAWINGS

FIG. 1 is a depiction of a bioengineered system for dermal applications. Inset - Image of an exemplary bioengineered device with a circular microneedle layer.
FIGS. 2A-2C are images of the appearance and flexibility of the epidermal bioengineered base later (FIGS. 2A and 2B) and the device having the appearance and coloration as the skin upon application (FIG. 2C).
FIG. 3 is a graph of the effect of silk fibroin solution concentration and ethanol processing duration of sample mechanical strength.
FIGS. 4A and 4B are graphs of ultimate tensile strength of silk base layers (FIG. 4A; mean ± SD, n=4/group) and elastic modulus of silk base layers at 0.25% strain (FIG. 4B; data calculated based on setting 0% strain when 0.05N of initial stress is achieved; data represent mean ± SD, n=4/group).
FIG. 5 is a graph of the effect of antioxidant conjugated hyaluronic acid (HAO) on human dermal fibroblast metabolic activity. The data is shown for D-methionine conjugated hyaluronic acid.
FIGS. 6A and 6B are images of histological evaluation (hematoxylin and eosin stained) of fibroblast ingrowth on silk fibroin only (FIG. 6A) and silk fibroin + antioxidant conjugated hyaluronic acid (FIG. 6B) base layers.
FIG. 7 is a graph of representative shear strength for chemically modified silk (carboxylated) at 90 minutes adhesion time.
FIGS. 8A and 8B are images of an exemplary device surface at 450X (FIG. 8A) and 1500X (FIG. 8B) magnification showing through-pores and partial pores.
FIGS. 9A-9B are scanning electron microscope mages of microneedle layers produced from silk fibroin at 50X (FIG. 9A) and 300X (FIG. 9B) magnification. FIG. 9C is an incident light image of an exemplary microneedle device at 30X magnification (FIG. 9C).
FIG. 10 is an image of the microneedle layer loaded with a fluorescent model drug (fluorescein).
FIG. 11 is a graph of the diffusion of a model drug (fluorescein) through the base layer after application onto the device surface.

### DETAILED DESCRIPTION OF THE INVENTION

The present disclosure is directed to devices formulated from natural biomaterials to feel and look like native tissue for use in regeneration in repair of the tissue and drug delivery to the tissue or underlying areas. The devices are biocompatible and fully assimilate as the new tissue regenerates. The devices comprise a base layer, which may allow adherence to the desired site without the need for sutures or other fixation methods, and, optionally, a microneedle layer, which facilitates local delivery of active agents (e.g., anesthetics, analgesics, and antibiotics) in the site of interest. As shown herein, the disclosed devices accelerate wound healing and tissue repair and regeneration processes.

### 1. Definitions

To facilitate an understanding of the present technology, a number of terms and phrases are defined below. Additional definitions are set forth throughout the detailed description.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

Unless otherwise defined herein, scientific, and technical terms used in connection with the present disclosure shall have the meanings that are commonly understood by those of ordinary skill in the art. The meaning and scope of the terms should be clear; in the event, however of any latent ambiguity, definitions provided herein take precedent over any dictionary or extrinsic definition. Further, unless otherwise required by context, singular terms shall include pluralities and plural terms shall include the singular.

A "subject" or "patient" may be human or non-human and may include, for example, animal strains or species used as "model systems" for research purposes, such a mouse model as described herein. Likewise, patient may include either adults, juveniles (e.g., children), or infants. Moreover, patient may mean any living organism, preferably a mammal (e.g., humans and non-humans) that may benefit from the administration of compositions contemplated herein. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish, and the like. In one embodiment, the mammal is a human.

The terms "contacting" or "applying" as used herein refers to bring or put in contact, to be in or come into contact, or apply to an area, thus referring to a state or condition of touching or of immediate or local proximity.

As used herein, the terms "providing," "administering," "introducing," are used interchangeably herein and refer to the placement of the disclosed device into a subject which results in at least partial localization of the device to a desired site.

As used herein, "bioresorbable" refers to a material that is susceptible to being chemically or enzymatically broken down into lower molecular weight chemical moieties by reagents and conditions that are naturally present in a biological environment. In an *in vivo* application, the chemical moieties may be assimilated into human or animal tissue, or otherwise removed from the point of implantation. A bioresorbable material that is "substantially completely" resorbed is highly resorbed (e.g., about 95% resorbed, or about 98% resorbed, or about 99% resorbed, or about 99.9% resorbed, or about 99.99% resorbed), but not completely (i.e., about 100%) resorbed. The disclosed device may be partially, substantially, or completely bioresorbed.

As used herein, "biocompatible" refers to a material that does not elicit an immunological rejection or detrimental effect when it is disposed within an *in vivo* biological environment. For example, a biological marker indicative of an immune response changes less than about 10%, or less than about 20%, or less than about 25%, or less than about 40%, or less than about 50% from a baseline value when a biocompatible material is implanted into a human or animal.

Preferred methods and materials are described below, although methods and materials similar or equivalent to those described herein can be used in practice or testing of the present disclosure. The materials, methods, and examples disclosed herein are illustrative only and not intended to be limiting.

### 2. Devices

Provided herein are devices comprising a base layer comprising a combination of silk fibroin and hyaluronic acid, wherein the hyaluronic acid is conjugated with an antioxidant, for use in tissue regeneration and delivery of active agents to a tissue of interest.

The devices disclosed herein are preferably biodegradable, biocompatible, and/or bioresorbable. In some embodiments, the device can entirely, substantially, or partially assimilate into a tissue for example, after 1-3 days, 1-3 weeks, 1-3 months or intermediate or greater periods. The desired duration may depend, for example, on the tissues involved and/or the composition of the device. In some embodiments, the device comprises a bioresorbable portion and a non-bioresorbable portion.

The devices may have a wide variety of sizes and shapes based on the intended tissue and area of coverage. The devices are, however, fairly thin to avoid unnecessary bulk when placed on or over tissues. The device according to the claimed invention is 0.5 mm to 5 mm thick. In some embodiments, the device is 0.5 mm thick, about 1 mm thick, about 1.5 mm thick, about 2 mm thick, about 2.5 mm thick, about 3 mm thick, about 3.5 mm thick, about 4 mm thick, about 4.5 mm thick, or 5 mm thick.

Given the devices are intended to be used topically on an exterior tissue surface, the devices preferably are substantially or entirely transparent during usage, or, alternatively or in addition, upon contact with the tissue (e.g., skin) have an appearance similar to that of the tissue. Furthermore, the devices have a level of flexibility consistent with the intended tissue or site of use.

The base layer comprises hyaluronic acid (HA). Hyaluronic acid, also known as hyaluronan or hyaluronate, is an anionic, non-sulfated glycosaminoglycan polymer comprising disaccharide units, which themselves include D-glucuronic acid and D-N-acetylglucosamine monomers, linked together via alternating β-1,4 and β-1,3 glycosidic bonds and pharmaceutically acceptable salts thereof. Hyaluronic acid can be purified from animal and non-animal sources. Polymers of hyaluronan can range in size from about 1,000 Da to about 20,000,000 Da. Any hyaluronic acid, or pharmaceutically acceptable salt thereof, is useful in the devices disclosed herein. Non-limiting examples of pharmaceutically acceptable salts of hyaluronic acid include sodium hyaluronan, potassium hyaluronan, magnesium hyaluronan, calcium hyaluronan, and combinations thereof.

The hyaluronic acid is conjugated (i.e. by a covalent bond) with at least one antioxidant. See for example, Kaderli, S., et al., Eur J Pharm Biopharm 2015;90:70-9.

The at least one antioxidant may include any antioxidant known in the art. Antioxidants include any man-made or natural substance which prevents or delays oxidative damage. Examples of antioxidants include, but are not limited to, vitamin A, vitamin C, vitamin E, selenium, and carotenoids (e.g., beta-carotene, lycopene, lutein, and zeaxanthin), glutathione, tocopherols, ubiquinol (coenzyme Q), amino acids (e.g., methionine, cysteine, tryptophan, tyrosine, homocysteine) or any combinations thereof. In some embodiments, the antioxidant comprises methionine, cysteine, tryptophan, tyrosine, homocysteine, Vitamin A, Vitamin C, Vitamin E, or a combination thereof. In particular embodiments, the antioxidant comprises methionine.

The hyaluronic acid may be of any molecular weight. The hyaluronic acid may be 1 kDa to 2 MDa In some embodiments, the hyaluronic acid may have a molecular weight less than about 250 kDa. The hyaluronic acid may have a molecular weight less than about 150 kDa, less than about 100 kDa, less than about 75 kDa, less than about 50 kDa, less than about 25 kDa, less than about 10 kDa, less than about 5 kDa or less than about 1 kDa. The hyaluronic acid may have a molecule weight greater than about 1 kDa, greater than about 5 kDa, greater than about 10 kDa, greater than about 25 kDa, greater than about 50 kDa, greater than about 75 kDa, greater than about 100 kDa, greater than about 150 kDa, or greater than about 200 kDa.

In some embodiments, the hyaluronic acid is high molecular weight hyaluronic acid. High molecular weight hyaluronic acid has a molecular weight greater than about 500 kDa. For example, high molecular weight hyaluronic acid may have a molecular weight between about 500 kDa and about 10 MDa. The hyaluronic acid may have a molecular weight less than 10 MDa, less than 8 MDa, less than 6 MDa, less than 4 MDa, less than 2 MDa, less than 1 MDa, less than 800 kDa, less than 700 kDa, less than 600 kDa, or less than 500kDa. The hyaluronic acid may have a molecular weight greater than 500 kDa, greater than 600 kDa, greater than 700 kDa, greater than 800 kDa, greater than 900 kDa, greater than 1MDa, greater than 2 MDa, greater than 4 MDa, greater than 6 MDa, or greater than 8 MDa.

The base layer may comprise from about 0.1% to about 10% w/v hyaluronic acid (HA). In some embodiments, the base layer comprises from about 0.1% w/v HA, about 0.5% w/v HA, about 1% w/v HA, about 2% w/v HA, about 3% w/v HA, about 4% w/v HA, about 5% w/v HA, about 6% w/v HA, about 7% w/v HA, about 8% w/v HA, about 9% w/v HA, or about 10% w/v HA.

In some embodiments, the base layer comprises from about 0.1% to about 0.5% w/v HA, about 0.1% to about 1% w/v HA, about 0.1% to about 2% w/v HA, about 0.1% to about 3% w/v HA, about 0.1% to about 4% w/v HA, about 0.1% to about 5% w/v HA, about 0.1% to about 6% w/v HA, about 0.1% to about 7% w/v HA, about 0.1% to about 8% w/v HA, about 0.1% to about 9% w/v HA, about 0.5% to about 1% w/v HA, about 0.5% to about 2% w/v HA, about 0.5% to about 3% w/v HA, about 0.5% to about 4% w/v HA, about 0.5% to about 5% w/v HA, about 0.5% to about 6% w/v HA, about 0.5% to about 7% w/v HA, about 0.5% to about 8% w/v HA, about 0.5% to about 9% w/v HA, about 0.5% to about 10% w/v HA, about 1% to about 2% w/v HA, about 1% to about 3% w/v HA, about 1% to about 4% w/v HA, about 1% to about 5% w/v HA, about 1% to about 6% w/v HA, about 1% to about 7% w/v HA, about 1% to about 8% w/v HA, about 1% to about 9% w/v HA, about 1% to about 10% w/v HA, about 2% to about 3% w/v HA, about 2% to about 4% w/v HA, about 2% to about 5% w/v HA, about 2% to about 6% w/v HA, about 2% to about 7% w/v HA, about 2% to about 8% w/v HA, about 2% to about 9% w/v HA, about 2% to about 10% w/v HA, about 3% to about 4% w/v HA, about 3% to about 5% w/v HA, about 3% to about 6% w/v HA, about 3% to about 7% w/v HA, about 3% to about 8% w/v HA, about 3% to about 9% w/v HA, about 3% to about 10% w/v HA, about 4% to about 5% w/v HA, about 4% to about 6% w/v HA, about 4% to about 7% w/v HA, about 4% to about 8% w/v HA, about 4% to about 9% w/v HA, about 4% to about 10% w/v HA, about 5% to about 6% w/v HA, about 5% to about 7% w/v HA, about 5% to about 8% w/v HA, about 5% to about 9% w/v HA, about 5% to about 10% w/v HA, about 6% to about 7% w/v HA, about 6% to about 8% w/v HA, about 6% to about 9% w/v HA, about 6% to about 1 0% w/v HA, about 7% to about 8% w/v HA, about 7% to about 9% w/v HA, about 7% to about 10% w/v HA, about 8% to about 9% w/v HA, about 8% to about 10% w/v HA, or about 9% to about 10% w/v HA.

According to the claimed invention, the base layer comprises a combination of hyaluronic acid and silk fibroin (SF).

In some embodiments, the silk fibroin is chemically or chemoenzymatically modified. A variety of chemical and chemoenzymatic modification of silk fibroin are known in the art. See for example, Kim SH, et al., Nat Commun 2018;9:1620, Simmons L, Tsuchiya K, Numata K. RSC Adv 2016;6:28737-44, and Chen J, Venkatesan H, Hu J. Adv Eng Mater 2018;20:1700961. Any of the known modifications may be suitable for use in the devices disclosed herein. In some embodiments, the chemically or chemoenzymatically modified silk fibroin comprises: carboxylated silk fibroin; hydroxylated silk fibroin; methylated silk fibroin; diazonium coupled silk fibroin; methacrylated silk fibroin; a silk fibroin modified at a tyrosine, hydroxy, or amine group or combinations thereof.

In some embodiments, the base layer comprises from about 0.1 to about 20% w/v silk fibroin. The base layer may comprise about 0.1% w/v, about 0.5% w/v, about 1% w/v, about 5% w/v, about 10% w/v, about 15% w/v, or about 20% w/v SF. In some embodiments, the base layer comprises from about 0.1% to about 0.5% w/v, about 0.1% to about 1% w/v, about 0.1% to about 5% w/v, about 0.1% to about 10% w/v, about 0.1% to about 15% w/v, about 0.5% to about 1% w/v, about 0.5% to about 5% w/v, about 0.5% to about 10% w/v, about 0.5% to about 15% w/v, about 0.5% to about 20% w/v, about 1% to about 5% w/v, about 1% to about 10% w/v, about 1% to about 15% w/v, about 1% to about 20% w/v, about 5% to about 10% w/v, about 5% to about 15% w/v, about 5% to about 20% w/v, about 10% to about 15% w/v, about 10% to about 20% w/v, or about 15% to about 20% w/v SF.

The base layer may further comprise other biocompatible and/or bioresorbable materials, including, but not limited to polytetrafluoroethylene (PTFE), polyurethane, polysulfone, cellulose and variants thereof, polyethylene, polypropylene, polyamide, polyester, polymethylmethacrylate, polylactic acid (PLA), polyglycolic acid (PGA), poly(lactic-co- glycolic acid) (PLGA), hydroxyapatite, polydioxanone (PDS), polycaprolactone (PCL), polyhydroxyalkanoate (PHA), polyglycerol sebacate (PGS), collagen, rice paper, and agarose or other hydrogels.

In some embodiments, the base layer further comprises a bioactive macromolecule. The bioactive macromolecule may comprise collagen, gelatin, fibrinogen, elastin, laminin, keratin, actin, myosin, cellulose, amylose, dextran, chitin, glycosaminoglycans, and combinations thereof.

In some embodiments, the base layer is from 0.5 mm to about 4 mm thick. The base layer may be 0.5 mm thick, about 1 mm thick, about 1.5 mm thick, about 2 mm thick, about 2.5 mm thick, about 3 mm thick, about 3.5 mm thick or about 4 mm thick.

In some embodiments, the base layer is self-adherent

In some embodiments, the device further comprises an adhesive layer. The adhesive layer may comprise any known biocompatible adhesive, including, but not limited to, polymeric adhesives, such as, polyacrylate polymers, rubber-based adhesives, and polysiloxane adhesives. In some embodiments, the adhesive layer comprises silk fibroin.

### a) Microneedle Layer

The device may further comprise a microneedle layer comprising a plurality of microneedles comprising a biocompatible material.

The microneedles may have any shape and/or dimension suitable for insertion into a tissue or across a biological barrier (e.g., skin). For example, the microneedles may fully or completely insert into the tissue, or a portion of the microneedle can be uninserted.

The microneedles may be from about 100 µm to about 2 mm in length. In some embodiments, the microneedles are about 100 µm, about 200 µm, about 300 µm, about 400 µm, about 500 µm, about 600 µm, about 700 µm, about 800 µm, about 900 µm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, or about 2 mm in length.

Each microneedle on the device need not have the same shape and/or dimension. In some embodiments, each microneedle is the same shape and/or dimension. In some embodiments, the device comprises two or more different types of microneedles, each having a defined shape or dimension.

In some embodiments, at least a portion or all of the microneedles are hollow. In some embodiments, at least a portion or all of the microneedles are at least partially filled.

The microneedles may be arranged in any pattern within the microneedle layer and the distance separating each microneedle in the microneedle layer may be varied based on the intended tissue or biological barrier target application.

The biocompatible material may include any of those materials known in the art, including those described and exemplified elsewhere herein. In some embodiments, the biocompatible material is selected from silk fibroin, hyaluronic acid, polyglycolic acid (PGA), polylactic acid (PLA), polydioxanone (PDS), polycaprolactone (PCL), poly(lactic-co-glycolic acid) PLGA, polyhydroxyalkanoate (PHA), and conjugates, variants, and combinations thereof. In some embodiments, the composition of the microneedle is the same or substantially the same as that of the base layer.

### b) Active Agent

The device may further comprise at least one active agent. The active agent may be a small molecule, a protein, an enzyme, a nucleic acid, a hormone, a steroid, an analgesic, an anesthetic, a vitamin, an antimicrobial agent, an anti-inflammatory agent, an antibody, or a combination thereof. In some embodiments, the active agent comprises an anesthetic, an analgesic, an antibiotic, or a combination thereof.

The active agent may be included throughout the device or in localized areas of the device. In some embodiments, the active agent is applied to an external surface of the microneedles. In some embodiments, the active agent is embedded throughout the base layer, the microneedle layer, or a combination thereof. For example, the active agent may diffuse through the pores of the base layer and or the microneedles to access the underlying tissue.

### c) Methods of Fabricating

Also disclosed herein are methods of manufacturing the devices described herein. The devices can be fabricated using a number of methods known in the art including micromachining, lithography, etching, three-dimensional printing, molding methods, or any combination of methods thereof or other methods known in the art for fabrication of similar devices. In some embodiments, the base layer may be fabricated using a different method from that of the microneedle device and/or the adhesive layer, when present. In some embodiments, the base layer and the microneedle device are fabricated using the same methods. In some embodiments, the base layer and the microneedle device are fabricated as one unit using a single method.

In some embodiments, the methods comprise: preparing a solution of the base layer components comprising a combination of silk fibroin and hyaluronic acid, wherein the hyaluronic acid is conjugated with an antioxidant; filling a base layer mold with the solution of the base layer components; removing a solidified base layer from the base layer mold; and crosslinking the base layer by treatment with 90% ethanol.

In some embodiments, the methods further comprise, filling a microneedle layer mold with a solution of the biocompatible material; removing a solidified microneedle layer from the microneedle layer mold; and adhering the base layer to the microneedle layer. The adhering can be accomplished using a number of methods know in the art. In some embodiments, the adhering comprises applying a solution of silk fibroin to one side of the base layer and or the microneedle layer; and joining the base layer to the microneedle layer.

In some embodiments, the methods comprise preparing a solution comprising a combination of silk fibroin and hyaluronic acid, wherein the hyaluronic acid is conjugated with an antioxidant; filling a combined microneedle-base layer mold with the solution; removing a solidified microneedle-base layer from the combined microneedle-base layer mold; and crosslinking the solidified microneedle-base layer by treatment with 90% ethanol.

The methods may further comprise adding an active agent In some embodiments, the active agent is added the solution of base layer components and/or the solution of biocompatible material, such that the active agent is homogenously mixed into the base layer, the microneedle layer, or both the base and microneedle layers.

In some embodiments, the active agent is added to the microneedle layer mold prior to filling with the biocompatible material. In some embodiments, the active agent is applied to at least a portion of the microneedle layer mold inner surface.

In some embodiments, the methods comprise forming the base layer and/or the microneedle layer using three-dimensional printing.

In some embodiments, both the base layer and the microneedle layer are formed using three-dimensional printing. In some embodiments, the base layer and the microneedle layer are three-dimensionally printed as a single object. Thus, the microneedle layer is 3D printed directly onto the base layer.

In some embodiments, the base layer or the microneedle layer are formed using a molding process. For example, in some embodiments, the base layer is 3D printed and the microneedle layer is formed in a negative mold. When the microneedle layer is manufacturing using a molding process, an active agent may be added to the mold as described above.

When the base layer and the microneedle layer are formed separately, the two layers can be adhered using methods known in the art. In some embodiments, the two layers are adhered by applying a solution of silk fibroin to one side of the base layer and or the microneedle layer; and joining the base layer to the microneedle layer.

The active agent may be added to the base layer, the microneedle layer, and/or the device after molding and fabrication. In some embodiments, a solution of the active agent is added to the base layer, the microneedle layer, and/or the device. In some embodiments, the active agent is added by applying a solution of the active agent on at least a portion of the base layer, the microneedle layer and/or the device. Thus, the active agent can diffuse into the base layer, the microneedle layer and/or the device due the porous nature of the compositions.

### 3. Methods of Use

### a) Methods of Tissue Regeneration and Repair

The disclosure also provides the disclosed devices for use in methods of regenerating and repairing a tissue, said methods comprising applying the disclosed devices to the tissue. Tissue regeneration and repair references processes to grow, renew or restore at least a portion of a tissue which has been damaged, lost, or diseased to return the tissue, at least partially, to its original structural, functional and physiological condition. In some embodiments, the tissue comprises a wound, an abrasion or loss of tissue, a burn, a suture, a cut, or any combination thereof.

The devices disclosed herein may modulate cell migration and proliferation, thereby reducing inflammation, accelerating wound healing, reduce scarring and ultimately promote repair, regeneration and restoration of structure and function in the tissue of interest. In some embodiments, the regeneration and repair comprise stimulation of fibroblast ingrowth into the tissue. In some embodiments, the regeneration and repair comprise increasing the metabolic activity of fibroblasts.

The tissue may be a soft tissue, including muscles, fibrous tissues, and fat. In some embodiments, the tissue comprises skin, muscle, fascia, or a subcutaneous tissue.

### b) Methods of Delivery of an Active Agent

Also described herein are the disclosed devices for use in methods for delivery of an active agent across a biological barrier, said methods comprising applying a device as disclosed herein to the biological barrier. A biological barrier can include any barrier to a tissue, including, but not limited to cell membranes, skin or layers thereof (e.g., epidermal and dermal tissue layers), other tissue layers (e.g., muscosal tissues, vascular tissues, and the like). In some embodiments, the biological barrier is the skin. In some embodiments, the biological barrier is the surface of a tissue.

Descriptions of devices and active agents set forth above is applicable to the disclosed methods.

### 4. Kits

Also within the scope of the present disclosure are kits that include devices described herein. In some embodiments, the kits comprise the devices described herein and an active agent. Descriptions of devices and active agents set forth above is also applicable to the kits.

Individual member components of the kits may be physically packaged together or separately. The components of the kit may be provided in bulk packages (e.g., multi-use packages) or single-use packages. The kits can also comprise instructions for using the components of the kit. The instructions are relevant materials or methodologies pertaining to the kit. The materials may include any combination of the following: background information, list of components and their availability information (purchase information, etc.), brief or detailed protocols for using the compositions, troubleshooting, references, technical support, and any other related documents. Instructions can be supplied with the kit or as a separate member component, either as a paper form or an electronic form which may be supplied on computer readable memory device or downloaded from an internet website, or as recorded presentation.

It is understood that the disclosed kits can be employed in connection with the disclosed methods. The kit may further contain additional containers or devices for use with the methods disclosed herein. The kits optionally may provide additional components such as wound dressings (gauze, adhesive bandages and the like), cotton swabs or wipes, and cleaning or antibiotic wipes.

The kits provided herein are in suitable packaging. Suitable packaging includes, but is not limited to, vials, bottles, jars, flexible packaging, and the like.

The following examples further illustrate the invention but should not be construed as in any way limiting its scope.

### EXAMPLES

### Materials and Methods

*Hyaluronic Acid (HA)-antioxidant:* HA can be used alone (reference-example, not according to the claimed invention) or (according to the claimed invention) chemically conjugated through various chemistries with antioxidants such as vitamin A, vitamin C, vitamin E, methionine, etc. as known in the art (See, Serban MA, Skardal A. Matrix Biology 2019;78-79 337-45). In a first step, HA was modified with chloroacetic acid to increase the conjugation sites of the polymer. Specifically, 0.8g of HA were dissolved in 8 mL of 45% w/v NaOH solution and magnetically stirred at room temperature for two hours before adding 60 mL of isopropanol and continuing to stir. Iodoacetic acid (20 mL solution of 0.432 M) was added to the HA/isopropanol mixture before covering with parafilm and stirring for 2 hours at room temperature. This mixture was filtered through a Buechner funnel (#2 Whatman filter paper) and the resulting carboxymethyl-HA (CMHA) cake was dissolved in 80 mL of DI water. The pH of the solution was adjusted to ~7.0 using 6N HCl. This CMHA solution was subsequently purified by dialysis against DI water (3500 MWCO dialysis cassettes) for 72 hours with 3-4 water changes per day to remove excess iodoacetic acid. The purified CMHA solution was removed from the cassettes, frozen at -80°C for ~3 hours or until fully frozen, and then lyophilized until completely dry. Next, antioxidants were covalently attached to CMHA using carbodiimide chemistry. Specifically, 25 mg of CMHA was dissolved into 5 mL of 2-(N-morpholino) ethanesulfonic acid (MES) buffer in a 50 mL beaker covered in parafilm with stir. The solution was mixed until CMHA was fully dissolved (about 25 minutes) and the antioxidant was added in a 1:2, 1:10 or 1:20 molar ratio, respectively, and allowed to mix until fully dissolved (about 5 minutes). A zero-length crosslinker [(1-ethyl-3-(3-dimethylaminopropyl) carbodiimide or EDC, 50 mg) was then added to the reaction mix and allowed to react for 20 hours. After 20 hours, the reaction was neutralized to a pH of 7.0 with an NaOH solution. The contents of the beaker were then added into dialysis cassettes and dialyzed against DI water, similarly to CMHA.

*Formation of SF*/*HA base layer:* A 135-155 mg/mL solution of silk fibroin (SF) was combined with a 40 mg/mL solution of HA (either unmodified (reference-example, not according to the claimed invention) or chemically modified with antioxidant, i.e. according to the claimed invention) and water to yield a series of varying mixes ranging from 60-120 mg/mL SF and 2-5 mg/mL HA component. A defined volume (2.2. mL) of this mix was cast into a 4°C pre-chilled 25x75x1 mm polydimethylsiloxane (PDMS) negative mold. This was then placed at -80°C until frozen, then lyophilized overnight to yield 1.0-1.2 mm thick base layers. These were then submerged in 90% EtOH for 24 hours to induce physical crosslinking then cut to desired size before being allowed to air dry.

*Formation of microneedle layer:* Microneedle template molds (500 µm needle height, 600 µm needle pitch) were epoxied to a three-dimensional (3D) printed base and used to cast PDMS negatives of the microneedle patch. SF solution alone or mixed with other macromolecules (60-80 mg/mL) was then placed into negative mold and allowed to dry for 24-48 hours at room temperature. The resulting microneedle layer was then submerged in 90% EtOH before allowing to air dry.

*Formation of microneedle and base layer assembly:* A microneedle layer and a base layer were briefly placed in deionized (DI) water to allow flexing and bending without breaking. A small paintbrush was used to apply a thin layer of 60-80 mg/mL silk fibroin solution to one side of the base layer and the microneedles were adhered to the base layer. This was allowed to air dry completely before crosslinking the silk adhesive for a few hours with 90% EtOH. If alcohol (methanol, ethanol, isopropanol, etc.) treatment was not applied, the assembled system is adherent.

*Base layer tension testing:* Base layers were cast and crosslinked as described above. A 3D printed dog bone-shaped stencil (40 mm length, 10 mm width in testing region) was used to cut out ~1 mm thick samples for tension testing on a DHR2 Rheometer. After crosslinking and drying, each tissue was briefly rinsed in DI water before allowing it to soak in phosphate buffered saline (PBS) for 30 min. Excess moisture was then dabbed off with a paper towel before loading the tissue into the tension testing fixture. A 20 mm loading gap was used and widened at a constant rate of 166.667 µm/sec.

### Cell Culture Experiments:

*MTS assay.* Primary adult fibroblasts were seeded at 1.5E4 cells/well in a 96-well plate, allowed to establish for 24 hours, then exposed to respective treatment in growth media for 24 hrs. Cell-titer aqueous one MTS assay was then used to determine metabolic activity relative to control.

*CyQuant assay.* These assays were set up identically to the above MTS assay, but rather than testing for cellular metabolic activity, cell viability/proliferation (based on the amount of DNA) was determined using the CyQuant NF assay kit

*Histology.* Base layers (~1 mm thick) were cast into cell culture inserts, lyophilized, then crosslinked/sterilized for 24 hours with 90% EtOH. The EtOH was allowed to dry off completely in sterile conditions then each tissue was pre-soaked for 2 hours in fibroblast growth media. primary adult fibroblasts were seeded at a density of 1E5 on the top of the base layers and allowed to grow submerged in growth media for 14 days before fixing, processing, sectioning, and staining with hematoxylin and eosin (H&E).

In the examples here below, only the examples including a base layer comprising a combination of silk fibroin and hyaluronic acid, wherein the hyaluronic acid is conjugated with an antioxidant, and wherein the device is treated with 90% ethanol to induce physical crosslinking, are according to the claimed invention. The other examples are to be considered as reference examples.

### Example 1

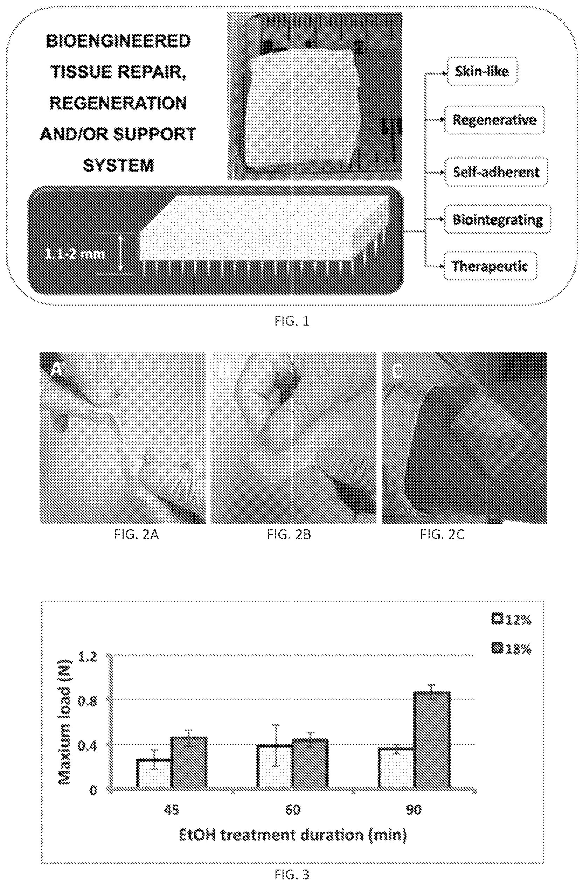

Bioengineered systems that mimic the natural skin feel and appearance were made of natural biomaterials such as silk fibroin (SF) and hyaluronic acid (HA) (FIGS. 2A-2C). In addition to SF, natural skin-specific macromolecules such as HA, collagen, elastin, laminin, fibronectin, etc. - either as natural macromolecules or chemically modified - can be incorporated into the SF devices in order to maximize their tissue-like properties.

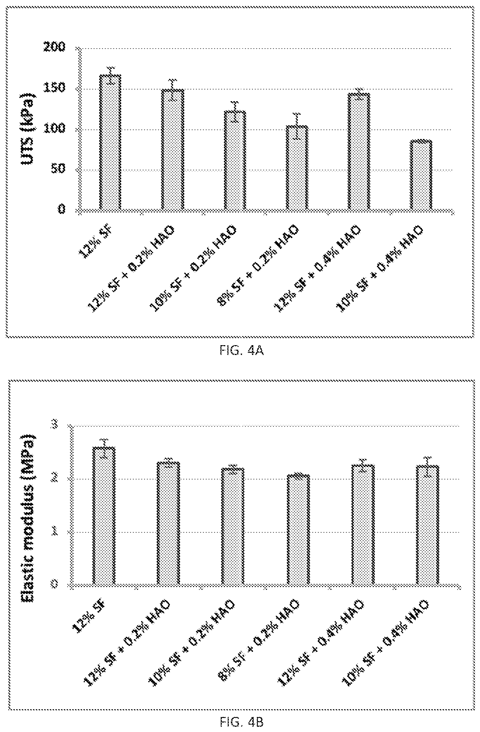

The mechanical properties of silk are partly responsible for its protective effects. By simply changing the biomaterial formulation or by altering the processing parameters, the mechanical properties of the skin like-devices can be tailored. Specifically, from a processing perspective, skin-like devices containing 12% and 18% w/v SF, respectively, were prepared and treated with 90% ethanol (EtOH) between 45-90 minutes. Subsequently samples were subjected to a tension test to evaluate their strength (FIG. 3). The results indicate a significant increase in sample strength with increased SF content and length of EtOH processing. From a formulation perspective, while keeping the processing parameters constant (24 hours 90% EtOH treatment) but varying the material composition, the mechanical properties of the constructs can be further customized (FIGS. 4A-4B).

### Example 2

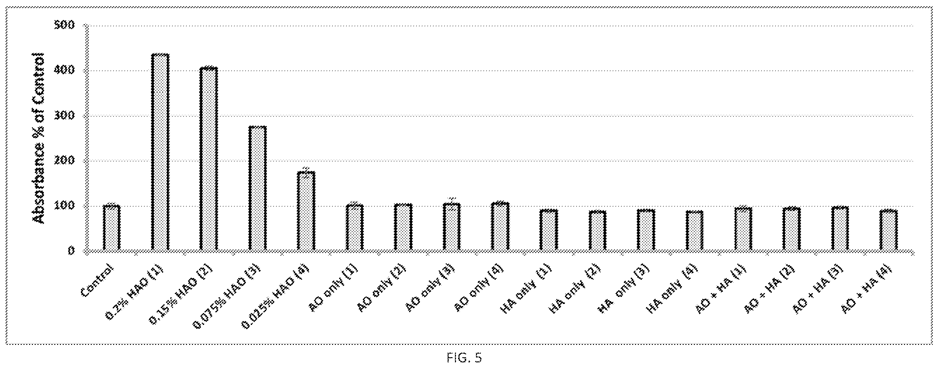

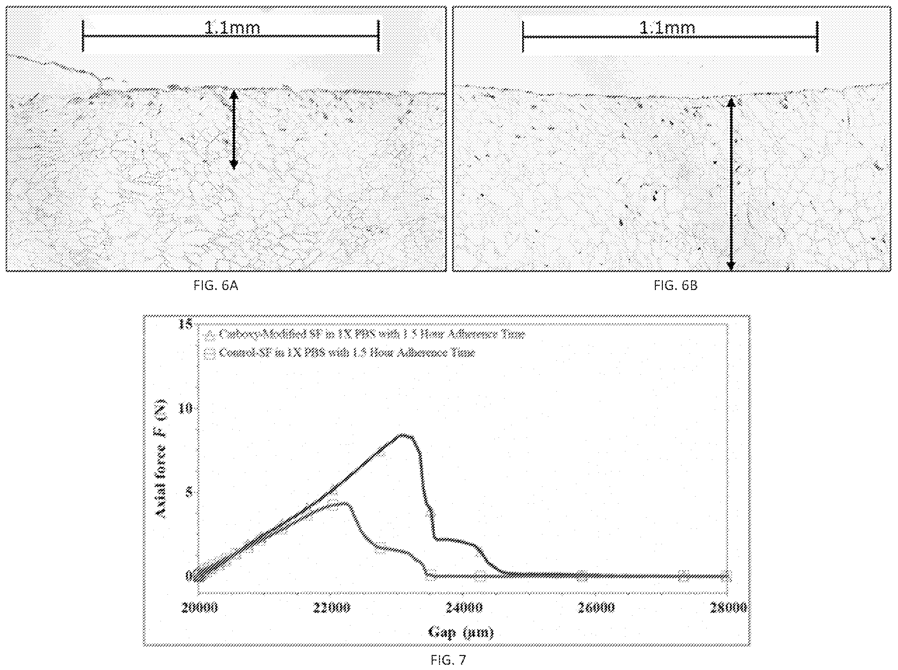

In response to a skin injury, wound healing occurs in three stages: inflammation, cellular proliferation and scar formation, and scar tissue remodeling. By chemically modifying HA with antioxidants to yield HA-antioxidant conjugates (HAO) the metabolic activity of primary human dermal fibroblasts was increased (FIG. 5). Use of antioxidant alone or antioxidant with HA but not conjugated did not produce a similar effect. Moreover, fibroblasts grew into constructs prepared with HAO to a higher extent compared to the SF only constructs (FIG. 6). Overall, these data indicate that exemplary devices, as disclosed herein can enhance wound healing and repair.

### Example 3

Currently available skin substitutes require fixation with sutures or staples and that increases the morbidity to the patient and procedure time for the medical personnel. On-contact adhesives entirely made of SF can be produced from SF solutions. By chemically modifying SF these adhesive properties can be further tailored (FIG. 7).

### Example 4

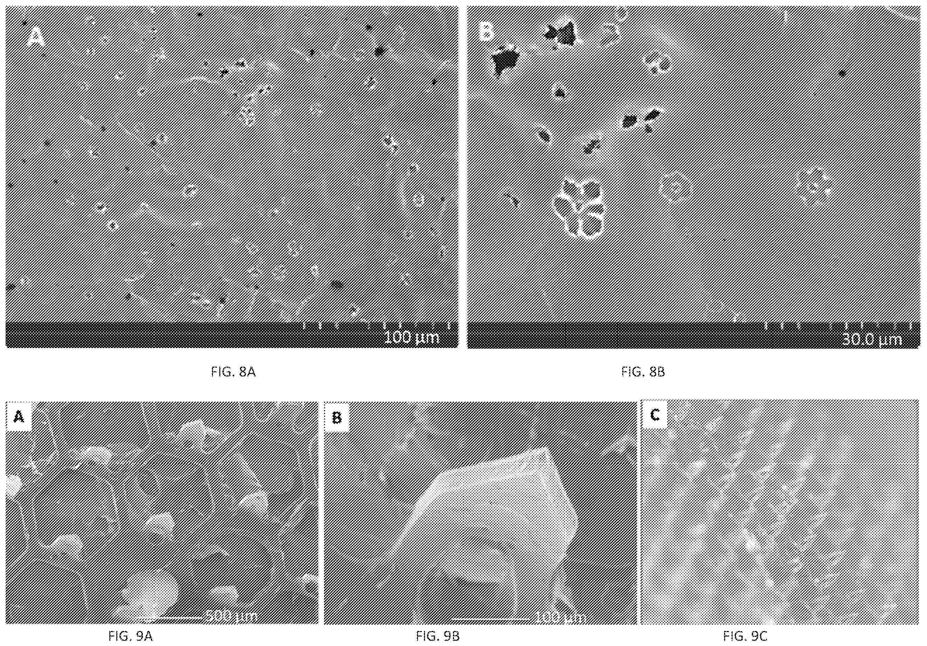

Patients that present with large surface wounds and especially with burns, are commonly treated with pain management medication such as opioids, and antibiotics for infection prevention. Systemic treatment of patients with opioids can lead to tolerance leading to dose escalation and ultimately dependence. The devices disclosed herein enable localized drug delivery to help minimize or eliminate the need for systemic drug administration, without the need for a drug to be actually incorporated into the system. Rather, these drugs would simple diffuse or pass though the bioengineered systems when applied to the surface. The surface architecture of the SF materials described in FIG. 2 was investigated by scanning electron microscopy (SEM). No pore-inducing agents (porogens) were used during the preparation and processing of these materials. The images revealed a micro-porous structure with opening in the range of 5-50 µm (FIG. 8A). Some of the porous structures have a funnel-like structure with one circular, narrower base and one wider, flower-like base (FIG. 8B). The porosity of the devices can be further customized via the use of porogens by following well-established protocols.

### Example 5

As mentioned above, the skin-like devices would, through their porous structure allow the passage of drugs to the underlaying tissues. These drugs could simple be applied as liquids on the surface of the device and would be expected to penetrate the device and reach the tissue. However, for optimal therapeutic effects, such drugs would ideally need to reach the dermis (tissue layer under the epidermis, that contains nerve endings and blood vessels) dermal layer of the skin. Although in many cases of severe injuries the dermis is exposed, the drug deployment properties the bioengineered system can be enhanced by incorporating microneedles into the device design. These microneedles would ensure the delivery of drugs to the dermis regardless of its exposure. By casting silk solutions into microneedle molds, high fidelity biomaterial microneedles were obtained (FIG. 9).

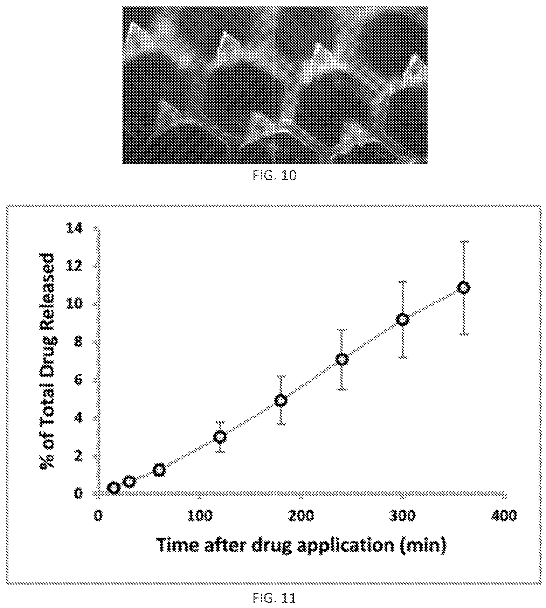

A drug or combination of drugs can be loaded onto or into the device for immediate or slow release upon placement at the wound site. This could be achieved either by placing the drug or drug solution into the microneedle mold, then casting the microneedles to have the drug on the microneedle surface (FIG. 10). Drugs could also be loaded into the base layer during or after formation, or into the microneedles by mixing into the casting solution. FIG. 11 shows the diffusion of fluorescein through the device, when applied onto the base layer. Alternatively, due to the porous structure of the base layer, drugs could be applied onto the device (base layer side) and would diffuse to the other side of the construct.

Preferred embodiments of this invention are described herein, including the best mode known to the inventors for carrying out the invention. Variations of those preferred embodiments may become apparent to those of ordinary skill in the art upon reading the foregoing description.

## Claims

1. A biocompatible device comprising a base layer comprising
a combination of silk fibroin and hyaluronic acid,
wherein the hyaluronic acid is conjugated with an antioxidant,
wherein the device is 0.5 mm to 5 mm thick and having a flexibility consistent with topical application on an exterior tissue surface such as skin,
and wherein the device is treated with 90% ethanol (EtOH) to induce physical crosslinking.

2. The device according to claim 1, wherein the antioxidant is selected from methionine, cysteine, tryptophan, tyrosine, homocysteine, Vitamin A, Vitamin C, and Vitamin E, or a combination thereof; and/or wherein the silk fibroin is chemically or chemoenzymatically modified, such as carboxylated silk fibroin, hydroxylated silk fibroin, methylated silk fibroin, diazonium coupled silk fibroin, methacrylated silk fibroin, a silk fibroin modified at a tyrosine, hydroxy, or amine group, or combinations thereof.

3. The device according to claim 1 or 2, wherein the base layer comprises from about 0.1% to about 20% w/v silk fibroin; and/or wherein the base layer comprises from about 0.1% to about 10% w/v hyaluronic acid.

4. The device according to any of claims 1-3, wherein the base layer further comprises a bioactive macromolecule selected from the group consisting of collagen, gelatin, fibrinogen, elastin, laminin, keratin, actin, myosin, cellulose, amylose, dextran, chitin, glycosaminoglycans, and combinations thereof.

5. The device according to any of claims 1-4, wherein the base layer is from 0.5 mm to about 4 mm thick.

6. The device according to any of claims 1-5, further comprising a microneedle layer comprising a plurality of microneedles comprising a biocompatible material, such as silk fibroin, hyaluronic acid, polyglycolic acid (PGA), polylactic acid (PLA), polydioxanone (PDS), polycaprolactone (PCL), poly(lactic-co-glycolic acid) PLGA, polyhydroxyalkanoate (PHA), and conjugates, and combinations thereof.

7. The device according to any of claims 1-6, wherein the device further comprises an adhesive layer comprising silk fibroin.

8. The device according to any of claims 1-7, wherein the device further comprises an active agent, such as, a protein, an enzyme, a nucleic acid, a hormone, a steroid, an analgesic, an anesthetic, a vitamin, an antimicrobial agent, an anti-inflammatory agent, an antibody, or a combination thereof, preferably an anesthetic, an analgesic, an antibiotic, or a combination thereof.

9. The device according to claim 8, wherein the active agent is (i) applied to an external surface of the microneedles, or (ii) embedded throughout the base layer, the microneedle layer, or a combination thereof.

10. A biocompatible device comprising a base layer comprising a combination of silk fibroin and hyaluronic acid, wherein the hyaluronic acid is conjugated with an antioxidant, and wherein the base layer is treated with 90% ethanol to induce crosslinking; for use in a method of regenerating and repairing a tissue, such as a wound, an abrasion or loss of tissue, a burn, a suture, a cut, or any combination thereof, such as skin, muscle, fascia, or a subcutaneous tissue; said method comprising applying a device comprising the base layer according to any of claims 1-9 to the tissue.

11. The biocompatible device for use according to claim 10, wherein the regenerating and repairing a tissue comprises stimulating fibroblast ingrowth into the tissue and increasing the metabolic activity of fibroblasts.

12. A method of fabricating the device according to any of claims 1-9, the method comprising:
preparing a solution of the base layer components comprising a combination of silk fibroin and hyaluronic acid, wherein the hyaluronic acid is conjugated with an antioxidant;
filling a base layer mold with the solution of the base layer components;
removing a solidified base layer from the base layer mold; and crosslinking the base layer by treatment with 90% ethanol.

13. The method according to claim 12, wherein :
(I) said method further comprises filling a microneedle layer mold with a solution of a biocompatible material; removing a solidified microneedle layer from the microneedle layer mold; applying a solution of silk fibroin to one side of the base layer and/or the microneedle layer; and joining the base layer to the microneedle layer; or
(II) the base layer mold is a combined microneedle-base layer mold, said method further comprises filling the combined microneedle-base layer mold with the solution; removing a solidified microneedle-base layer from the combined microneedle-base layer mold; and crosslinking the solidified microneedle-base layer.

14. The method according to claim 13, wherein the method further comprises adding an active agent to
(i) the microneedle layer mold prior to filling with the biocompatible material, such as by applying the active agent to a least a portion of the microneedle layer mold inner surface; , the biocompatible material, or a combination thereof;
(ii) the combined microneedle-base layer mold prior to filling with the solution, such as by applying the active agent to a least a portion of the combined microneedle-base layer mold inner surface; or
(iii) the base layer, the microneedle layer, the device, or any combination thereof, such as by
soaking the base layer, the microneedle layer, the device, or any combination thereof with a solution of the active agent or pouring a solution of the active agent on the base layer, the microneedle layer, the device, or any combination thereof.

## Patentansprüche

1. Biokompatible Vorrichtung, umfassend eine Basisschicht, umfassend
eine Kombination aus Seidenfibroin und Hyaluronsäure,
wobei die Hyaluronsäure mit einem Antioxidans konjugiert ist,
wobei die Vorrichtung 0,5 mm bis 5 mm dick ist und eine Flexibilität aufweist, die mit einer topischen Anwendung auf einer äußeren Gewebeoberfläche wie beispielsweise der Haut vereinbar ist,
und wobei die Vorrichtung mit 90%igem Ethanol (EtOH) behandelt ist,um eine physikalische Vernetzung zu induzieren.

2. Vorrichtung nach Anspruch 1, wobei das Antioxidans ausgewählt ist aus Methionin, Cystein, Tryptophan, Tyrosin, Homocystein, Vitamin A, Vitamin C und Vitamin E oder einer Kombination davon und/oder wobei das Seidenfibroin chemisch oder chemoenzymatisch modifiziert, wie beispielsweise carboxyliertes Seidenfibroin, hydroxyliertes Seidenfibroin, methyliertes Seidenfibroin, diazoniumgekoppeltes Seidenfibroin, methacryliertes Seidenfibroin, ein an einer Tyrosin-, Hydroxy- oder Aminogruppe modifiziertes Seidenfibroin oder Kombinationen davon, ist.

3. Vorrichtung nach Anspruch 1 oder 2, wobei die Basisschicht von etwa 0,1 % bis etwa 20 % w/v Seidenfibroin umfasst und/oder wobei die Basisschicht von etwa 0,1 % bis etwa 10 % w/v Hyaluronsäure umfasst.

4. Vorrichtung nach einem der Ansprüche 1-3, wobei die Basisschicht weiter ein bioaktives Makromolekül umfasst, ausgewählt aus der Gruppe, bestehend aus Kollagen, Gelatine, Fibrinogen, Elastin, Laminin, Keratin, Aktin, Myosin, Cellulose, Amylose, Dextran, Chitin, Glykosaminoglykanen und Kombinationen davon.

5. Vorrichtung nach einem der Ansprüche 1-4, wobei die Basisschicht von 0,5 mm bis etwa 4 mm dick ist.

6. Vorrichtung nach einem der Ansprüche 1-5, weiter umfassend eine Mikronadelschicht, die eine Vielzahl von Mikronadeln umfasst, umfassend ein biokompatibles Material wie beispielsweise Seidenfibroin, Hyaluronsäure, Polyglykolsäure (PGA), Polymilchsäure (PLA), Polydloxanon (PDS), Polycaprolacton (PCL), Poly(milchsäure-co-glykolsäure) (PLGA), Polyhydroxyalkanoat (PHA) und Konjugate sowie Kombinationen davon.

7. Vorrichtung nach einem der Ansprüche 1-6, wobei die Vorrichtung weiter eine Klebeschicht aus Seidenfibroin umfasst.

8. Vorrichtung nach einem der Ansprüche 1-7, wobei die Vorrichtung weiter einen Wirkstoff wie beispielsweise ein Protein, ein Enzym, eine Nukleinsäure, ein Hormon, ein Steroid, ein Analgetikum, ein Anästhetikum, ein Vitamin, ein antimikrobielles Mittel, ein entzündungshemmendes Mittel, einen Antikörper oder eine Kombination davon, vorzugsweise ein Anästhetikum, ein Analgetikum, ein Antibiotikum oder eine Kombination davon, umfasst.

9. Vorrichtung nach Anspruch 8, wobei der Wirkstoff (i) auf eine äußere Oberfläche der Mikronadeln aufgebracht oder (ii) überall in die Basisschicht, die Mikronadelschicht oder eine Kombination davon eingebettet ist.

10. Biokompatible Vorrichtung, umfassend eine Basisschicht, umfassend eine Kombination von Seidenfibroin und Hyaluronsäure, wobei die Hyaluronsäure mit einem Antioxidans konjugiert ist und die Basisschicht mit 90%igem Ethanol behandelt ist,um eine Vernetzung zu induzieren; zur Verwendung in einem Verfahren zum Regenerieren und Reparieren eines Gewebes, wie beispielsweise einer Wunde, einer Abschürfung oder einem Gewebeverlust, einer Verbrennung, einer Naht, eines Schnitts oder einer Kombination davon, wie beispielsweise Haut, Muskeln, Faszien oder eines Unterhautgewebes;
wobei das Verfahren das Aufbringen einer Vorrichtung, welche die Basisschicht nach einem der Ansprüche 1-9 umfasst, auf das Gewebe umfasst.

11. Biokompatible Vorrichtung zur Verwendung nach Anspruch 10, wobei das Regenerieren und Reparieren eines Gewebes das Stimulieren des Einwachsens von Fibroblasten in das Gewebe und das Steigern der Stoffwechselaktivität von Fibroblasten umfasst.

12. Verfahren zum Herstellen der Vorrichtung nach einem der Ansprüche 1-9, wobei das Verfahren Folgendes umfasst:
Herstellen einer Lösung der Basisschichtkomponenten, umfassend eine Kombination von Seidenfibroin und Hyaluronsäure, wobei die Hyaluronsäure mit einem Antioxidans konjugiert ist;
Befüllen einer Basisschichtform mit der Lösung der Basisschichtkomponenten;
Entnehmen einer verfestigten Basisschicht aus der Basisschichtform; und
Vernetzen der Basisschicht durch Behandlung mit 90%igem Ethanol.

13. Verfahren nach Anspruch 12, wobei:
(I) das Verfahren weiter das Befüllen einer Mikronadelschichtform mit einer Lösung eines biokompatiblen Materials; das Entnehmen einer verfestigten Mikronadelschicht aus der Mikronadelschichtform; das Aufbringen einer Seidenfibroinlösung auf eine Seite der Basisschicht und/oder die Mikronadelschicht; und das Verbinden der Basisschicht mit der Mikronadelschicht umfasst; oder
(II) die Basisschichtform eine kombinierte Mikronadel-Basisschichtform ist, wobei das Verfahren weiter das Befüllen der kombinierten Mikronadel-Basisschichtform mit der Lösung; das Entnehmen einer verfestigten Mikronadel-Basisschicht aus der kombinierten Mikronadel-Basisschichtform; und das Vernetzen der verfestigten Mikronadel-Basisschicht umfasst.

14. Verfahren nach Anspruch 13, wobei das Verfahren weiter das Hinzufügen eines Wirkstoffs umfasst zu
(i) der Mikronadelschichtform vor dem Befüllen mit dem biokompatiblen Material, wie beispielsweise durch Aufbringen des Wirkstoffs auf mindestens einen Abschnitt der Innenfläche der Mikronadelschichtform, das biokompatible Material oder eine Kombination davon;
(ii) der kombinierten Mikronadel-Basisschichtform vor dem Befüllen mit der Lösung, wie beispielsweise durch Aufbringen des Wirkstoffs auf mindestens einen Abschnitt der Innenfläche der kombinierten Mikronadel-Basisschichtform; oder
(iii) der Basisschicht, der Mikronadelschicht, der Vorrichtung oder einer beliebigen Kombination davon, wie beispielsweise durch Tränken der Basisschicht, der Mikronadelschicht, der Vorrichtung oder einer beliebigen Kombination davon mit einer Lösung des Wirkstoffs oder durch Gießen einer Lösung des Wirkstoffs auf die Basisschicht, die Mikronadelschicht, die Vorrichtung oder eine beliebige Kombination davon.

## Revendications

1. Dispositif biocompatible comprenant une couche de base comprenant
une combinaison de fibroïne de soie et d'acide hyaluronique,
dans lequel l'acide hyaluronique est conjugué à un antioxydant,
dans lequel le dispositif a une épaisseur de 0,5 mm à 5 mm et présentant une flexibilité compatible avec une application topique sur une surface de tissu extérieure telle que de la peau,
et dans lequel le dispositif est traité avec 90 % d'éthanol (EtOH) pour induire une réticulation physique.

2. Dispositif selon la revendication 1, dans lequel l'antioxydant est sélectionné parmi la méthionine, cystéine, tryptophane, tyrosine, homocystéine, vitamine A, vitamine C et vitamine E, ou une combinaison de celles-ci ; et/ou dans lequel la fibroïne de soie est modifiée chimiquement ou chimioenzymatiquement, comme la fibroïne de sole carboxylée, fibroïne de soie hydroxylée, fibroïne de soie méthylée, fibroïne de soie couplée au diazonium, fibroïne de soie méthacrylatée, fibroïne de soie modifiée au niveau d'un groupe tyrosine, hydroxy ou amine, ou des combinaisons de celles-ci.

3. Dispositif selon la revendication 1 ou 2, dans lequel la couche de base comprend d'environ 0,1 % à environ 20 % p/v de fibroïne de sole ; et/ou dans lequel la couche de base comprend d'environ 0,1 % à environ 10 % p/v d'acide hyaluronique.

4. Dispositif selon l'une quelconque des revendications 1-3, dans lequel la couche de base comprend en outre une macromolécule bioactive sélectionnée à partir du groupe constitué de collagène, gélatine, fibrinogène, élastine, laminine, kératine, actine, myosine, cellulose, amylose, dextrane, chitine, glycosaminoglycanes et combinaisons de ceux-ci.

5. Dispositif selon l'une quelconque des revendications 1-4, dans lequel la couche de base a une épaisseur de 0,5 mm à environ 4 mm.

6. Dispositif selon l'une quelconque des revendications 1-5, comprenant en outre une couche à microaiguilles comprenant une pluralité de microaiguilles comprenant un matériau biocompatible, tel que la fibroïne de soie, acide hyaluronique, acide polyglycolique (PGA), acide polylactique (PLA), polydioxanone (PDS), polycaprolactone (PCL), poly(acide lactique-co-glycolique) PLGA, polyhydroxyalcanoate (PHA), et conjugués, et leurs combinaisons.

7. Dispositif selon l'une quelconque des revendications 1-6, dans lequel le dispositif comprend en outre une couche adhésive comprenant de la fibroïne de soie.

8. Dispositif selon l'une quelconque des revendications 1-7, dans lequel le dispositif comprend en outre un agent actif, tel qu'une protéine, une enzyme, un acide nucléique, une hormone, un stéroïde, un analgésique, un anesthésique, une vitamine, un agent antimicrobien, un agent anti-inflammatoire, un anticorps ou une combinaison de ceux-ci, de préférence un anesthésique, un analgésique, un antibiotique ou une combinaison de ceux-ci.

9. Dispositif selon la revendication 8, dans lequel l'agent actif est (i) appliqué sur une surface externe des microaiguilles, ou (ii) incorporé à travers la couche de base, la couche à microaiguilles ou une combinaison de celles-ci.

10. Dispositif biocompatible comprenant une couche de base comprenant une combinaison de fibroïne de soie et d'acide hyaluronique, dans lequel l'acide hyaluronique est conjugué à un antioxydant, et dans lequel la couche de base est traitée avec de l'éthanol à 90 % pour induire une réticulation ; destiné à être utilisé dans un procédé de régénération et de réparation d'un tissu, tel qu'une plaie, une abrasion ou une perte de tissu, une brûlure, une suture, une coupure, ou toute combinaison de celles-ci, tels que de la peau, du muscle, du fascia ou un tissu sous-cutané ;
ledit procédé comprenant l'application d'un dispositif comprenant la couche de base selon l'une quelconque des revendications 1-9 sur le tissu.

11. Dispositif biocompatible destiné à être utilisé selon la revendication 10, dans lequel la régénération et la réparation d'un tissu comprennent la stimulation de la croissance des fibroblastes dans le tissu et l'augmentation de l'activité métabolique des fibroblastes.

12. Procédé de fabrication du dispositif selon l'une quelconque des revendications 1-9, le procédé comprenant :
la préparation d'une solution des composants de couche de base comprenant une combinaison de fibroïne de soie et d'acide hyaluronique, dans lequel l'acide hyaluronique est conjugué à un antioxydant ;
le remplissage d'un moule de couche de base avec la solution des composants de couche de base ;
le retrait d'une couche de base solidifiée du moule de couche de base ; et
réticulation de la couche de base par traitement à l'éthanol à 90 %.

13. Procédé selon la revendication 12, dans lequel :
(I) ledit procédé comprend en outre le remplissage d'un moule à couche à microaiguilles avec une solution d'un matériau biocompatible ; le retrait d'une couche à microaiguilles solidifiée du moule de couche à microaiguilles ; et l'application d'une solution de fibroïne de soie sur un côté de la couche de base et/ou la couche à microaiguilles ; et la jonction de la couche de base à la couche à microaiguilles ; ou
(II) le moule de couche de base est un moule de couche de base à microaiguilles combiné, ledit procédé comprend en outre le remplissage du moule de couche de base à microaiguilles combiné avec la solution ; le retrait d'une couche de base à microaiguilles solidifiée du moule de couche de base à microaiguilles combiné ; et la réticulation de la couche de base à microaiguilles solidifiée.

14. Procédé selon la revendication 13, dans lequel le procédé comprend en outre l'ajout d'un agent actif
(i) au moule de couche à microaiguilles avant le remplissage avec le matériau biocompatible, tel qu'en appliquant l'agent actif sur au moins une portion de la surface intérieure de moule de couche à microaiguilles, le matériau biocompatible, ou une combinaison de ceux-ci ;
(ii) au moule de couche de base à microaiguilles combiné avant le remplissage avec la solution, tel qu'en appliquant l'agent actif sur au moins une portion de la surface intérieure de moule de couche de base à microaiguilles combiné ; ou
(iii) à la couche de base, la couche à microaiguilles, le dispositif ou toute combinaison de ceux-ci, tel qu'en trempant la couche de base, la couche à microaiguilles, le dispositif ou toute combinaison de ceux-ci avec une solution de l'agent actif ou en versant une solution de l'agent actif sur la couche de base, la couche à microaiguilles, le dispositif ou toute combinaison de ceux-ci.
